# EUROPEAN PATENT APPLICATION

(11) **EP 3 824 886 A1**
(43) Date of publication of application: **26.05.2021**
(21) Application number: 19837097.5
(22) Date of filing: 15.07.2019
(51) Int. Cl.: A61K 31/231, A61K 35/32, A61K 31/201, A23L 33/10, A61P 29/00, A61P 35/00

(54) **NOVEL COMPOUNDS ISOLATED FROM CERVI PARVUM CORNU, AND PHARMACEUTICAL USES THEREOF**

(30) Priority: 16.07.2018 KR 20180082265
(71) Applicant: FNG Research Co., Ltd., Cheongju-si, Chungcheongbuk-do 28357 (KR)
(72) Inventor: GOO, Young Sam, Hongseong-gun Chungcheongnam-do 32292 (KR); SON, Ki Nam, Cheongju-si Chungcheongbuk-do 28407 (KR)
(74) Representative: Danubia Patent & Law Office LLC
(86) International application number: PCT/KR2019/008736
(87) International publication number: WO 2020/017852

(57) **Abstract**

The present disclosure provides a pharmaceutical composition for the prevention or treatment of inflammatory diseases, leukopenia or neutropenia and a method for synthesizing the same, the pharmaceutical composition including as an active ingredient at least one selected from compounds represented by the Formulas 1 to 4.

## Description

### TECHNICAL FIELD

The present disclosure relates to a novel compound isolated from *Cervi Parvum Cornu* and pharmaceutical uses thereof, and further, a chemical synthesis method of the novel compound.

### BACKGROUND ART

*Cervi Parvum Cornu* is one obtained by cutting and drying the non-ossified horn of deer, such as *Cervus nippon, Cervus elaphus,* or *Cervus canadensis,* which belongs to the family Cervidae.

*Cervi Parvum Cornu* is traditionally widely used as a Chinese herbal medicine that represents supplements. According to the literature "Donguibogam", it is known that *Cervi Parvum Cornu* has a great variety of effects, including tonic, hematic, aphrodisiac, analgesic, hematopoietic, growth promotion, heart failure treatment and function increasing effects, as well as the effects of recovering from fatigue, enhancing the vital energy of the body and enhancing the urination function of the kidneys.

It is known that *Cervi Parvum Cornu* contains various free amino acids and polysaccharides, glycosaminoglycans (GAGs), hyaluronic acid, keratin, sialic acid, cholesterol, fatty acids, phospholipids, mineral components and the like.

As a method of taking *Cervi Parvum Cornu,* a method of taking a filtrate by extracting *Cervi Parvum Cornu* with hot water together with various kinds of herbal medicines, or a method of taking it as a pill after crushing it with medicinal herbs and making it into a powder is mainly used, but various studies have been conducted to extract and isolate physiologically active components from *Cervi Parvum Cornu* using solvent extraction and fractionation methods.

Korean Patent Publication No. 1999-0044781 discloses that *Cervi Parvum Cornu* (*Cervus nippon*) is extracted with chloroform, and the chloroform extract is fractionated using silica gel column chromatography to separate five types of monoacetyldiacylglycerol compounds, among which 1-palmitoyl-2-linoleoyl-3-acetyl glycerol represented by the following Formula (PLAG, hereinafter referred to as "PLA glycerol") has proliferation-promoting activity of hematopoietic stem cells and platelet progenitor cells. Since then, in relation to the research on the PLA glycerol, Korean Patent Publication No. 2006-0047447 discloses immunomodulators and anticancer agents, Korean Patent Publication No. 2015-0021464 discloses a composition for inhibiting blood cancer or cancer metastasis, Korean Patent Publication No. 2015-0021465 discloses a composition for preventing or treating rheumatoid arthritis, and Korean Patent Publication No. 2017-0005484 discloses a composition for treating leukopenia and thrombocytopenia.

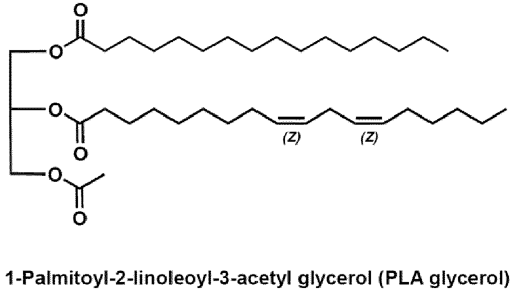

Meanwhile, Korean Patent Publication No. 2000-0059468 discloses that *Cervi Parvum Cornu* (*Cervus nippon*) is extracted with ethanol, and the ethanol extract is fractionated using silica gel column chromatography, thereby obtaining the structures of four types of new phospholipid-based compounds including a compound of the following Formula and their antifungal activity.

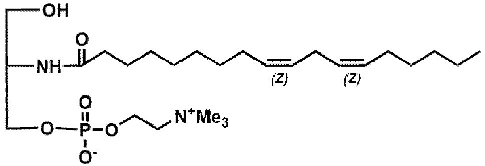

*Cervi Parvum Cornu* has various pharmacological activities as described above, and its ingredients are also very diverse, Therefore, there is a need for continuous research on pharmacologically active components that are not yet known.

### DISCLOSURE OF INVENTION

### TECHNICAL PROBLEM

It is an object of the present disclosure to isolate a novel compound having anti-inflammatory physiological activity from *Cervi Parvum Cornu* using solvent extraction and fractionation methods, and chemically synthesize the compound so as to enable stable mass production, thereby providing a composition for the prevention or treatment of inflammatory diseases, and a composition for the prevention or treatment of leukopenia or neutropenia.

### SOLUTION TO PROBLEM

One embodiment of the present disclosure provides a pharmaceutical composition for the prevention or treatment of inflammatory diseases, which comprises as an active ingredient at least one selected from compounds represented by the following Formulas 1 to 4 by analyzing the active ingredient isolated from *Cervi Parvum Cornu.*

Another embodiment of the present disclosure provides a composition for the prevention or treatment of leukopenia and neutropenia, comprising as an active ingredient at least one selected from compounds represented by the following Formulas 1 to 4.

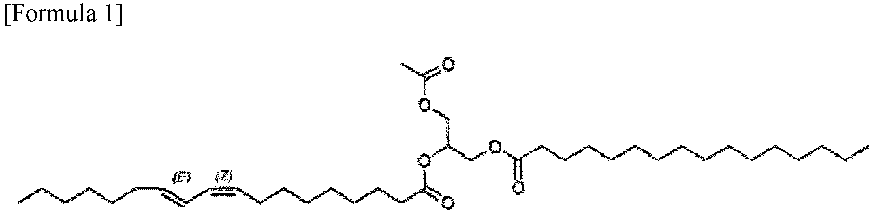

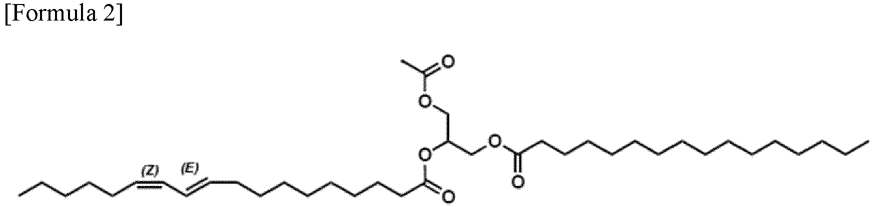

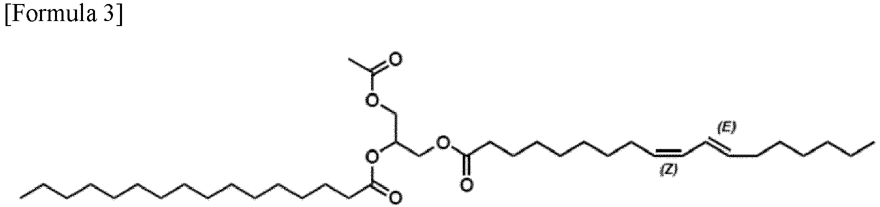

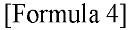

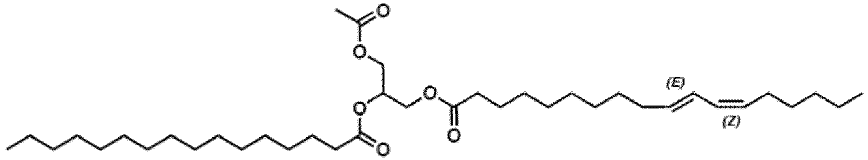

The compound represented by Formula 1 and the compound represented by Formula 2 are isomers of each other, and the compound represented by Formula 3 and the compound represented by Formula 4 are also isomers of each other.

The compound according to the present disclosure can be used for the prevention or treatment of inflammatory disease caused by inflammatory cytokines, for example, inflammatory diseases selected from the group consisting of atopic dermatitis, edema, dermatitis, allergy, asthma, conjunctivitis, periodontitis, rhinitis, otitis media, sore throat, tonsillitis, pneumonia, gastritis, colitis, gout, hepatic spondylitis, fibromyalgia, psoriatic arthritis, osteoarthritis, rheumatoid arthritis, shoulder periarthritis, myositis, hepatitis, cystitis, and nephritis.

The compound according to the present disclosure can be used for the prevention or treatment of antineoplastic chemotherapy-induced neutropenia (CIN).

Yet another embodiment of the present disclosure provides a method for synthesizing 1-palmitoyl-2-conjugated linoleoyl-3-acetyl glycerol (PCA glycerol) comprising the steps of: (a) reacting palmitic acid with a compound represented by the following Formula 5 under basic conditions to synthesize 1-palmitoyl glycerol; (b) reacting the 1-palmitoyl glycerol with acetyl halide under basic conditions to synthesize 1-palmitoyl-3-acetyl glycerol; and (c) reacting the 1-palmitoyl-3-acetyl glycerol with conjugated linoleic acid under basic conditions.

A further embodiment of the present disclosure provides a method for synthesizing 1-conjugated linoleoyl-2-palmitoyl-3-acetyl glycerol (CPA glycerol) comprising the steps of: (a) reacting conjugated linoleic acid with a compound represented by the following Formula (5) under basic conditions to synthesize 1-conjugated linoleoyl-glycerol; (b) reacting the 1-conjugated linoleoyl-glycerol with acetyl halide under basic conditions to synthesize 1-conjugated linoleoyl-3-acetyl glycerol; and (c) reacting the 1-conjugated linoleoyl-3-acetyl glycerol with palmitic acid under basic conditions.

The conjugated linoleic acid used in the synthesis may be conjugated linoleic acid (cis-9, trans-11) or conjugated linoleic acid (trans-10, cis-12) alone or a mixture thereof.

### ADVANTAGEOUS EFFECTS

The novel compounds of Examples 1 and 2 according to the present disclosure remarkably inhibited the production of representative inflammatory cytokines IL-6, IL-1β and TNF-α and also remarkably inhibited neutropenia caused by anticancer drugs, as compared with a control group. In addition, it was confirmed that they showed significant inhibitory effects on cytokine production and neutropenia even in comparison with PLA glycerol of Comparative Example. Therefore, they can be effectively used as a composition for preventing, ameliorating or treating inflammatory diseases according to the present disclosure, and they showed significant inhibitory effects on antineoplastic chemotherapy-induced neutropenia (CIN), and thus, can be used as a prophylactic or therapeutic agent thereof.

### BRIEF DESCRIPTION OF THE DRAWINGS

FIG. 1 a diagram showing an extraction method using three organic solvents from *Cervi Parvum Cornu* according to the present disclosure.
FIG. 2 is a diagram showing a method for fractionating extract C2 extracted from *Cervi Parvum Cornu* according to the present disclosure.
FIG. 3 is a mass spectrometry data of the fraction C2-2-E-a-Ms according to the present disclosure.
FIG. 4 is an LC data of the fraction C2-2-E-a-Ms according to the present disclosure.
FIG. 5(A) is the UV analysis data of the fraction C2-2-E-a-Ms according to the present disclosure, (B) is the UV analysis data of PLA glycerol of Comparative Example, (C) is the UV analysis data of conjugated linoleic acid, and (D) is the UV analysis data of linoleic acid.
FIG. 6 is a graph showing the results of measuring cell viability by the MTT assay of the novel compounds of Examples 1 and 2 of the present disclosure.
FIG. 7 is a graph showing the IL-6 production-inhibiting effect of the novel compounds of Examples 1 and 2 of the present disclosure.
FIG. 8 is a graph showing the IL-1β production-inhibiting effect of the novel compounds of Examples 1 and 2 of the present disclosure.
FIG. 9 is a graph showing the TNF-α inhibitory effect of the novel compounds of Examples 1 and 2 of the present disclosure.
FIG. 10 is a graph showing the NO production inhibitory effect of the novel compounds of Examples 1 and 2 of the present disclosure.
FIG. 11 is a graph showing the inhibitory effect of the novel compounds of Examples 1 and 2 of the present disclosure on neutropenia induced by an anticancer agent (gemcitabine).
FIG. 12 is a graph showing the inhibitory effect of the novel compounds of Examples 1 and 2 of the present disclosure on neutropenia induced by an anticancer agent (tamoxifen).

### BEST MODE FOR CARRYING OUT THE INVENTION

The present inventors analyzed and carried out the solvent extraction method and the fractionation method of the prior literatures from various angles in order to develop a pharmaceutical composition for the prevention or treatment of inflammatory diseases derived from *Cervi Parvum Cornu,* isolated a novel compound having excellent physiological activity against inflammatory diseases by the following method, confirmed that the novel compound has a significant inhibitory effect on neutropenia (CIN) induced by chemotherapy drugs, developed a novel method for synthesizing the compound, and completed the present disclosure.

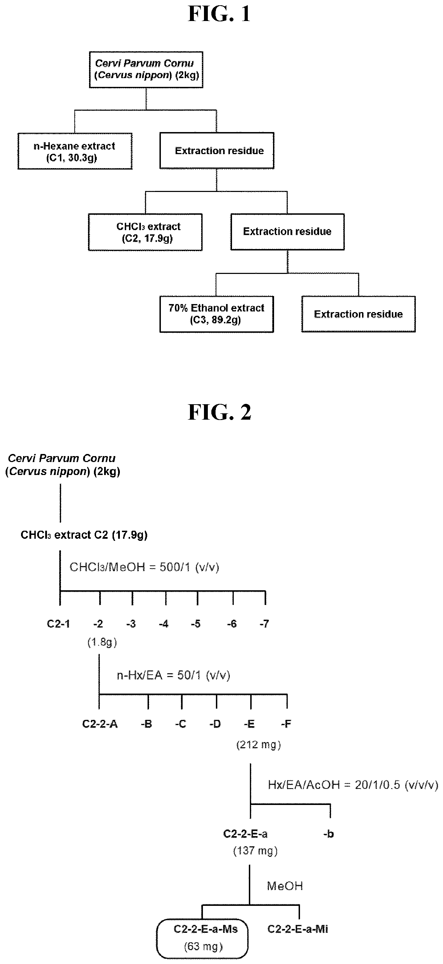

In order to extract the physiologically active component from *Cervi Parvum Cornu, Cervi Parvum Cornu* (*Cervus nippon*) was extracted sequentially using three organic solvents of hexane, chloroform, and 70% ethanol as shown in FIG. 1, and then subjected to a silica gel column chromatography and a thin layer liquid chromatography as shown in FIG. 2 to isolate a novel compound having excellent anti-inflammatory activity.

### Extraction using organic solvent

2kg of *Cervus nippon* purchased on the market was pulverized, placed in a beaker, to which 9L of hexane was added as the primary extraction solvent, and then the mixture was heated at 80°C for 2 hours, extracted twice, and filtered to obtain a hexane extract. The hexane extract was distilled under reduced pressure and dried to obtain 30.3 g of extract C1.

After the hexane extraction, 9 L of chloroform (CHCl₃) was added as a secondary extraction solvent to the remaining residue, and then heated at 80°C for 2 hours, extracted twice, and filtered to obtain a chloroform extract. The chloroform extract was distilled under reduced pressure and dried to obtain 17.9 g of extract C2.

After the chloroform extraction, 9L of 70% ethanol was added as a tertiary extraction solvent to the remaining residue, and then heated at 80°C for 5 hours, extracted twice, and filtered to obtain an ethanol extract. The ethanol extract was distilled under reduced pressure and dried to obtain 89.2 g of extract C3.

As a result of anti-inflammatory activity experiments for each of the extracts C1, C2 and C3 obtained above, it was confirmed that the extract C2 exhibited high anti-inflammatory activity effects, and the physiologically active component was fractionated from the extract C2 by silica gel column chromatography.

### Silica gel column chromatography

A mixed solution of CHCl₃/MeOH (500:1, v/v) was added to powder silica gel and swelled, and then packed in an open column. 17.9 g of the obtained extract C2 was dissolved in CHCl₃/MeOH (500:1, v/v) and subjected to a column packed with a silica gel. While allowing CHCl₃/MeOH (500:2, v/v) as an eluent to flow through the column, the eluted fractions were collected by 50 ml and then developed by TLC (developing solvent: CHCl₃/MeOH=300:1), and stained with iodine (I₂), and then seven main fractions were isolated based on Rf values. Each of the isolated fractions was distilled under reduced pressure and dried to obtain fractions C2-1 to C2-7. As a result of the anti-inflammatory activity experiments for the seven fractions, it was confirmed that the fraction C2-2 exhibited high anti-inflammatory activity effects, and the physiologically active component was fractionated again on the fraction C2-2 using silica gel column chromatography.

1.8 g of the fraction C2-2 was taken. 50 ml of a mixed solution of hexane (n-Hx)/ethyl acetate (EA) (50: 1) was added to 20 g of powder silica gel, swelled, and packed in the column. 1.8 g of the fraction C2-2 was dissolved in a minimum amount of n-Hx/EA (50:1, v/v) as an eluent and subjected to a silica gel column. While allowing the eluent to flow through the silica gel column, the eluted fractions were collected by 15 ml, developed by TLC (developing solvent: n-Hx/EA=50:1, v/v), stained with iodine, and then the six main fractions were isolated based on Rf values. Each of the isolated fractions were distilled under reduced pressure and dried to obtain fractions C2-2-A to C2-2-F. As a result of the anti-inflammatory activity experiments for the seven fractions, it was confirmed that the fraction C2-2-E exhibited high anti-inflammatory activity effects, and the physiologically active component was fractionated again on the fraction C2-2-E using silica gel column chromatography.

212 mg of the fraction C2-2-E was taken. 10 ml of a mixed solution of n-Hx/EA/AcOH (20:1:0.5, v/v/v) was added to 3.5 g of powder silica gel, swelled, and packed in the column. 212 mg of the fraction C2-2-E was dissolved in n-Hx/EA/AcOH (20:1:0.5, v/v/v) as an eluent and subjected to a silica gel column. While allowing the eluent to flow through the silica gel column, the eluted fractions were collected by 10 ml and developed by TLC (developing solvent: n-Hx/EA/AcOH=20:1:0.5, v/v/v), stained with iodine, and then the two main fractions were isolated based on Rf values. Each of the isolated fractions was distilled under reduced pressure and dried to obtain fractions C2-2-E-a and C2-2-E-b, respectively. As a result of the anti-inflammatory activity experiments for the two fractions, it was confirmed that the fraction C2-2-Ea exhibited high anti-inflammatory activity effects, and the physiologically active component was fractionated again on the fraction C2-2-E using silica gel column chromatography.

137 mg of the fraction C2-2-E-a was taken. 5 ml of methanol was added to the fraction C2-2-Ea, mixed, and isolated into a methanol-soluble fraction C2-2-Ea-Ms and a methanol-insoluble fraction C2-2-Ea-Mi, and each fraction was distilled under reduced pressure, and dried to obtain 63 mg of the fraction C2-2-Ea-Ms. As a result of the anti-inflammatory activity experiments for the two fractions, it was confirmed that the fraction C2-2-E-a-Ms exhibited high anti-inflammatory activity effects.

Anti-inflammatory inhibition evaluation of the fractions was performed by the method according to Experimental Example 2 described later, and the results are shown in Table 1 below.

**[Table 1]**

| Fraction (100µg/ml) | IL-6 production amount (pg/ml) | IL-1β production amount (pg/ml) | TNF-α production amount (pg/ml) |
|---|---|---|---|
| C2-2 | 453 | 59 | 283 |
| C2-2-E | 402 | 43 | 233 |
| C2-2-E-a | 321 | 36 | 197 |
| C2-2-E-a-Ms | 253 | 34 | 162 |

As shown in Table 1, it was confirmed that as the fraction was smaller, the production of inflammatory cytokines IL-6, IL-1β, and TNF-α was more inhibited. In order to confirm the active component of the fraction C2-2-E-a-Ms, which has the highest inflammation inhibitory activity among the fractions, component analysis was performed using a MALDI-TOF/TOF mass spectrometer (Bruker UltraflextremeTM, German), liquid chromatography (LC) and UV analyzer.

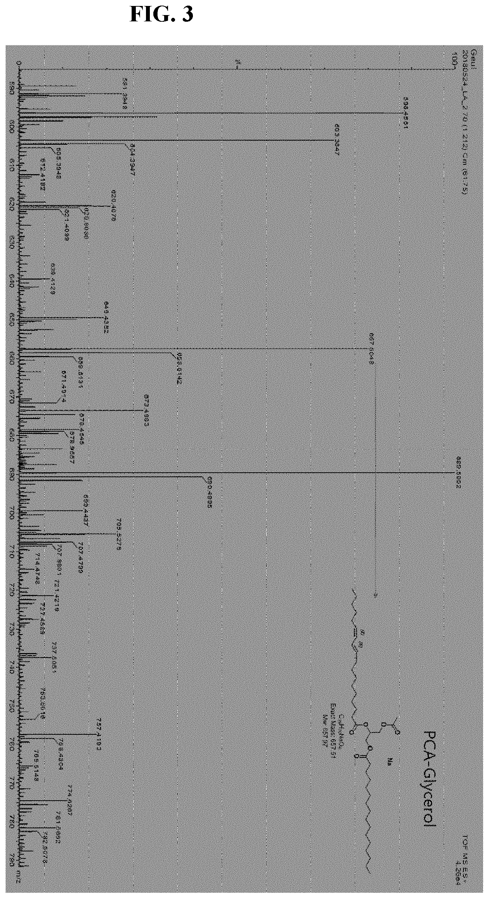

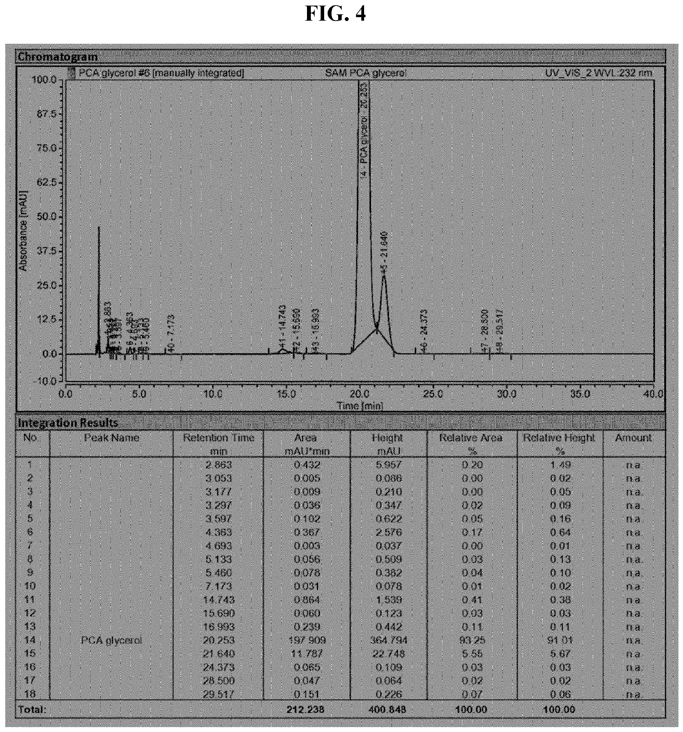

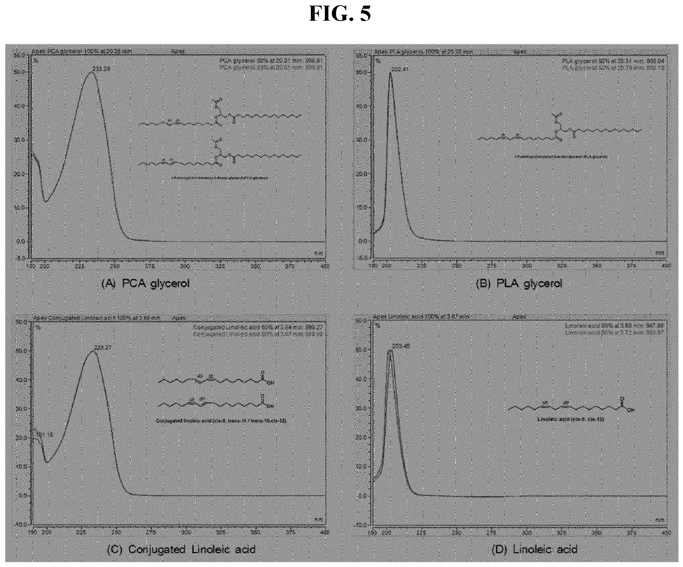

FIG. 3 is a mass spectrometry data of the fraction C2-2-E-a-Ms according to the present disclosure. FIG. 4 is an LC data of the fraction C2-2-E-a-Ms according to the present disclosure. In FIG. 5, (A) is the UV analysis data of the fraction C2-2-E-a-Ms according to the present disclosure, (B) is the UV analysis data of PLA glycerol of Comparative Example, (C) is the UV analysis data of conjugated linoleic acid, and (D) is the UV analysis data of linoleic acid.

As a result of analyzing the mass of the active ingredient of the fraction C2-2-E-a-Ms of the present disclosure, the mass was the same as that of the known PLA glycerol isolated from *Cervi Parvum Corn* (Compound KJ-3 of Korean Patent Publication No. 1999-0044781). However, as a result of the UV analysis, it showed a UV analysis pattern completely different from that of PLA glycerol. Comparing the UV pattern of (C) conjugated linoleic acid and the UV pattern of (D) linoleic acid in FIG. 5, it was confirmed that the compound of the fraction C2-2-Ea-Ms of the present disclosure has a novel structure of conjugated linoleoyl.

The anti-inflammatory physiologically active component according to the present disclosure are novel compounds represented by the following Formulas 1 to 4 in which acetyl, palmitoyl, and conjugated linoleoyl are bonded to a glycerol structure. This was confirmed for the first time in the present disclosure

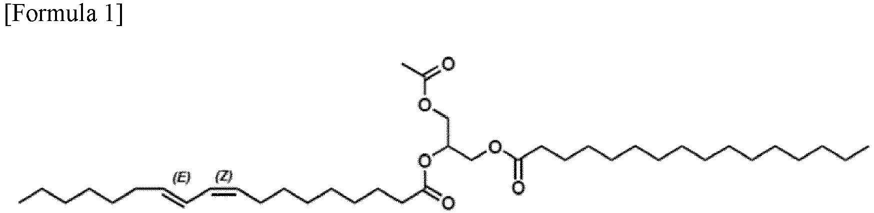

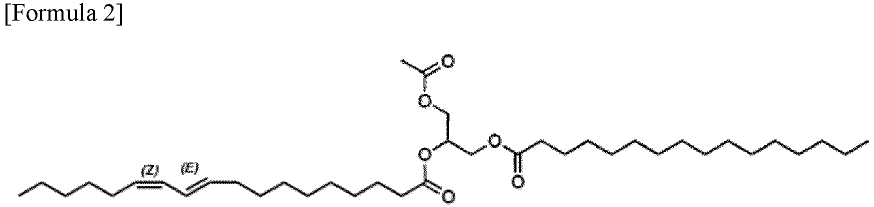

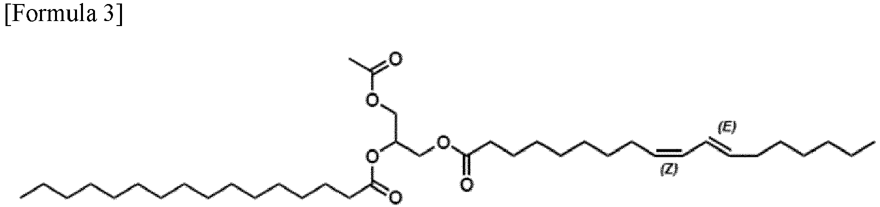

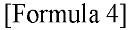

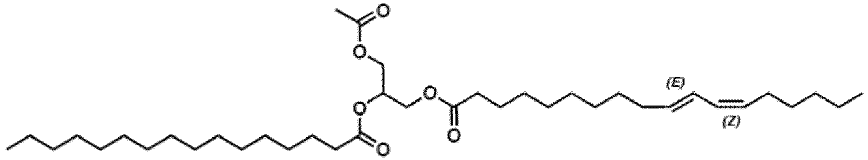

### Chemical synthesis of PCA glycerol represented by Formula 1 and Formula 2

The compounds of Formulae 1 and 2 (PCA glycerol) according to the present disclosure can be prepared in high yield according to the following Reaction Scheme 1.

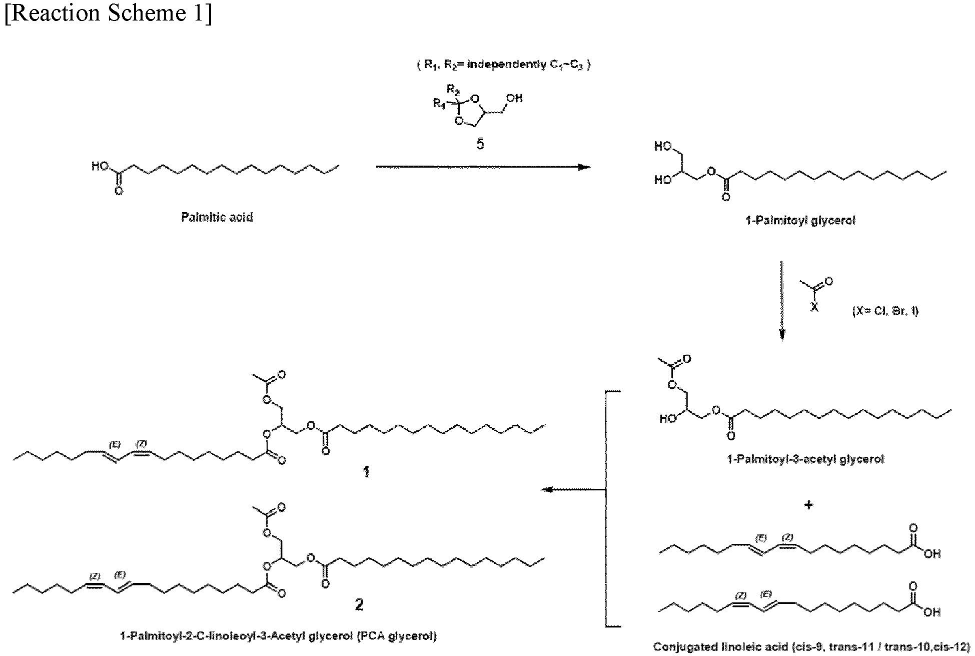

The synthesis method of PCA glycerol according to the present invention includes the steps of:
(a) reacting palmitic acid with a compound represented by the following Formula 5 under basic conditions to synthesize 1-palmitoyl glycerol;
(b) reacting the 1-palmitoyl glycerol with acetyl halide under basic conditions to synthesize 1-palmitoyl-3-acetyl glycerol; and
(c) reacting the 1-palmitoyl-3-acetyl glycerol with conjugated linoleic acid under basic conditions to synthesize 1-palmitoyl-2-conjugated linoleoyl-3-acetyl glycerol (PCA glycerol).

In step (a), the compound of Formula 5 is a glycerol derivative protected by a 1,3-diol compound. The reaction may be carried out under basic conditions such as trimethylamine. In order to maximize the reaction activity of palmitic acid, the reaction can be carried out via an anhydride reaction with the addition of pivaloyl halide. When deprotected with hydrochloric acid or the like after the reaction, 1-palmitoyl glycerol is synthesized. The equivalent weight (eq.) of palmitic acid and the compound of Formula 5 is preferably 0.9:1.1 to 1.1:0.9, but is not limited thereto.

In step (b), the equivalent weight of 1-palmitoyl glycerol and acetyl halide is preferably 1:1 to 1:1.6, but is not limited thereto.

In step (c), in order to maximize the reaction activity of the conjugated linoleic acid, the reaction can be carried out via anhydride reaction with the addition of pivaloyl halide. The equivalent weight (eq.) of 1-palmitoyl-3-acetyl glycerol and conjugated linoleic acid is preferably 0.9:1.1 to 1.1:0.9, but is not limited thereto.

Meanwhile, the compounds of Chemcical Formulae 3 and 4 (CPA glycerol) according to the present disclosure may be prepared in high yield according to Reaction Formula 2 below.

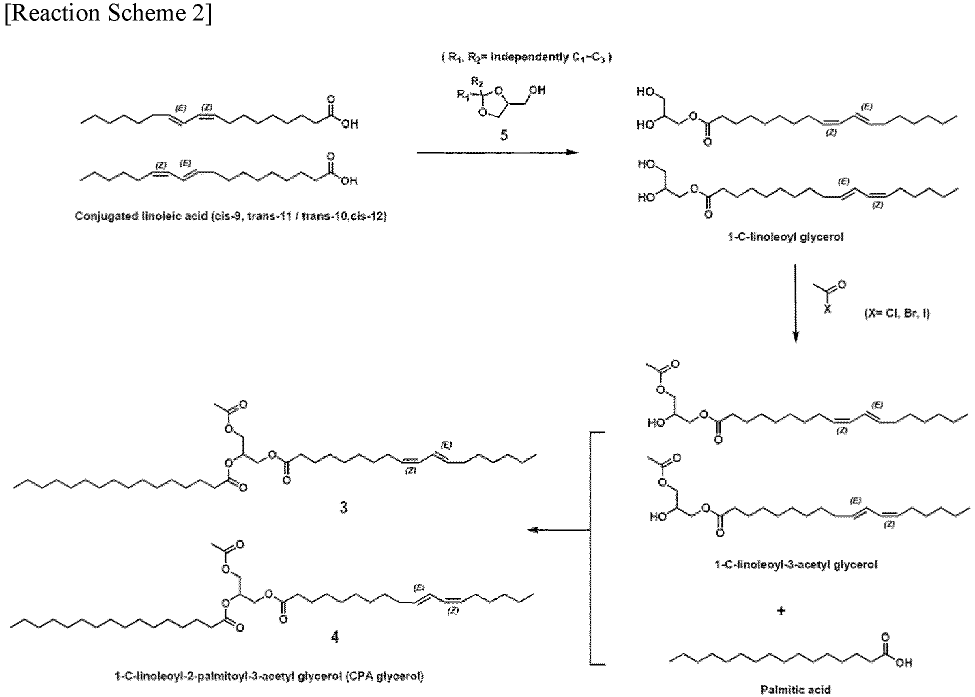

The synthesis method of PCA glycerol according to the present invention includes the steps of:
(a) reacting conjugated linoleic acid with a compound represented by the following Formula 5 under basic conditions to synthesize 1-conjugated linoleoyl-glycerol;
(b) reacting the 1-conjugated linoleoyl-glycerol with acetyl halide under basic conditions to synthesize 1-conjugated linoleoyl-3-acetyl glycerol; and
(c) reacting the 1-conjugated linoleoyl-3-acetyl glycerol with palmitic acid under basic conditions to synthesize 1-conjugated linoleoyl-2-palmitoyl-3-acetyl glycerol (CPA glycerol). In steps (a) and (c), pivaloyl halide may be added for the anhydride reaction.

Hereinafter, a method for synthesizing the novel compound of the present disclosure will be described in detail by way of examples.

### Example 1: Preparation of 1-Palmitoyl-2-conjugated-linoleoyl-3-acetyl glycerol (PCA glycerol)

### (1) Preparation of 1-Palmitoyl glycerol

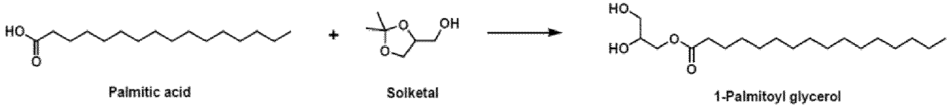

20.0 g of palmitic acid and 17.0 g of triethylamine were added to 200 ml of methylene chloride (MC), cooled to 0°C, and then 10.0 g of pivaloyl chloride was slowly added dropwise, and then stirred for 2 hours, to which 10.8 g of Solketal and 0.1 g of 4-dimethylaminopyridine were added, and the mixture was stirred at room temperature for 12 hours. 200 ml of purified water was added, the layers were separated and then the organic layer was concentrated in vacuo. 200 ml of methanol and 20 ml of hydrochloric acid were added to the concentrated mother liquor, and stirred at room temperature for 10 hours. 300 ml of n-hexane and 300 ml of purified water were added thereto, stirred for 3 hours, filtered and dried in vacuo to give 21.9 g (yield: 85.0%) of the target compound.

### (2) Preparation of 1-Palmitoyl-3-acetyl glycerol

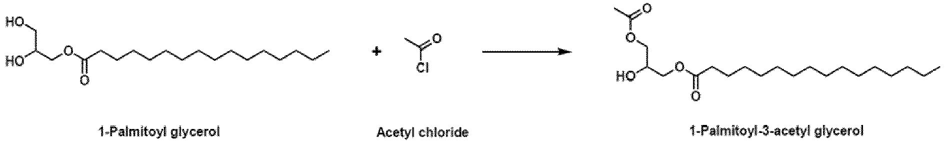

20.0 g of the obtained 1-palmitoyl glycerol, 33.5 g of pyridine, and 0.2 g of 4-dimethylaminopyridine were added to 200 ml of MC, and stirred for 1 hour, to which 7.1 g of acetyl chloride was added dropwise and stirred for 5 hours. 200 ml of purified water was added thereto and neutralized with dilute hydrochloric acid. The layers were separated and the organic layer was dehydrated with MgSO₄ and concentrated in vacuo. 100 ml of n-hexane was added thereto, crystallized at 10°C or lower, filtered, and then dried in vacuum to give 18.5 g (yield: 82.0%) of the target compound.

### (3) Preparation of 1-Palmitoyl-2-C-linoleoyl-3-acetyl glycerol (PCA glycerol)

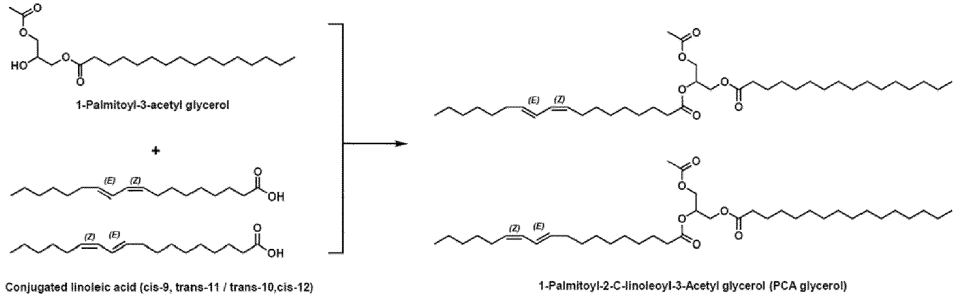

14.0 g of conjugated linoleic acid (cis-9,trans-11/trans-10,cis-12) and 10.8 g of triethylamine were added to 180ml of n-hexane, cooled to 0°C, and 7.0 g of pivaloyl chloride was slowly added dropwise thereto. After completion of the dropwise addition, the mixture was stirred at the same temperature for 1 hour. 18.0 g of 1-palmitoyl-3-acetyl glycerol and 1.0 g of 4-dimethylaminopyridine were added thereto and then stirred at room temperature for 10 hours. 180 ml of purified water was added, the layers were separated and the organic layer was dehydrated with MgSO₄, concentrated in vacuo, and purified by silica gel column (eluent: n-Hx:EA=20:1, v/v) to give 26.1 g of the target compound (yield: 85%).

### Example 2: Preparation of 1-Conjugated-linoeoyl-2-palmitoyl-3-acetyl glycerol (CPA glycerol)

### (1) Preparation of 1-C-linoleoyl glycerol

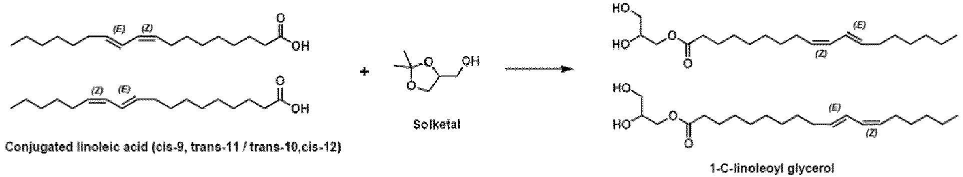

22.0 g of conjugated linoleic acid (cis-9,trans-11/trans-10,cis-12) and 10.8 g of triethylamine were added to 200ml of n-hexane, cooled to 0°C, and 7.0 g of pivaloyl chloride was slowly added dropwise and stirred at the same temperature for 1 hour. Then, 10.8 g of Solketal and 0.1 g of 4-dimethylaminopyridine were added, and the mixture was stirred at room temperature for 12 hours. 200 ml of purified water was added, the layers were separated and the organic layer was concentrated in vacuo. 200 ml of methanol and 20 ml of hydrochloric acid were added to the concentrated mother liquor and stirred at room temperature for 10 hours. 300ml of n-hexane and 300ml of purified water were added, stirred for 3 hours, filtered, and dried in vacuo to give 22.8g (yield: 82%) of the target compound.

### (2) Preparation of 1-C-linoleoyl-3-acetyl glycerol

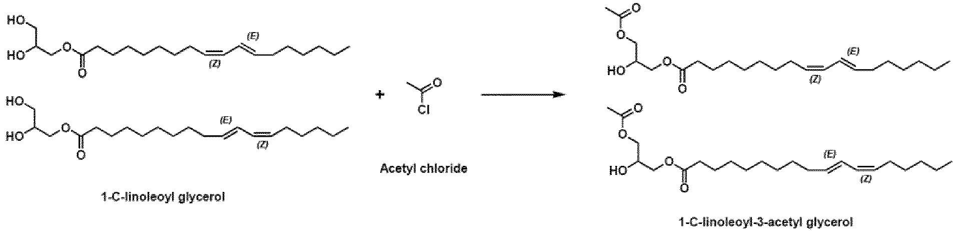

20.0g of 2-C-linoleoyl glycerol, 33.5g of pyridine and 0.2g of 4-dimethylaminopyridine were added to 200ml of MC, and stirred for 1 hour. 6.6 g of acetyl chloride was added dropwise and stirred for 5 hours. 200 ml of purified water was added, neutralized with diluted hydrochloric acid, the layers were separated and the organic layer was dehydrated with MgSO₄ and concentrated in vacuo. 100ml of n-hexane was added thereto and crystallized at 10°C or lower, filtered and then dried in vacuo to give 17.7 g (yield: 79.0%) of the target compound.

### (3) Preparation of 1-C-linoleoyl-2-palmitoyl-3-acetyl glycerol (CPA glycerol)

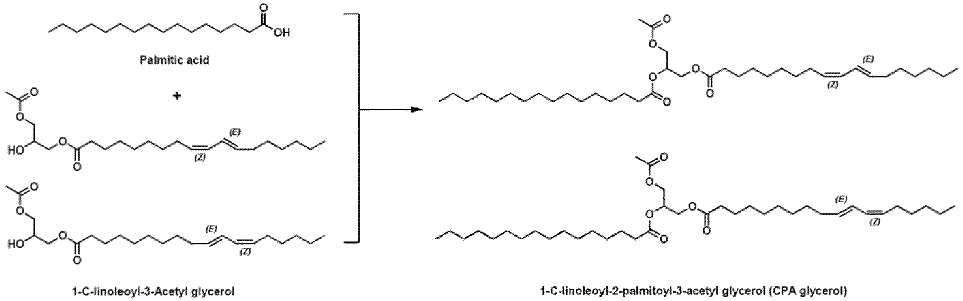

12.2g of palmitic acid and 10.8g of triethylamine were added to 180ml of n-hexane, cooled to 0°C, and 7.0g of pivaloyl chloride was slowly added dropwise thereto. After completion of the dropwise addition, the mixture was stirred at the same temperature for 1 hour, and then 18.0 g of 1-C-linoleoyl-3-acetyl glycerol and 1.0 g of 4-dimethylaminopyridine were added and then stirred at room temperature for 10 hours. 180 ml of purified water was added, the layers were separated and the organic layer was dehydrated with MgSO₄, concentrated in vacuo, and purified by a silica gel column (eluent: n-Hx:EA=20:1, v/v) to give 25.9 g (yield: 86%) of the target compound.

### Comparative Example: Preparation of 1-Palmitoyl-2-linoleoyl-3-acetyl glycerol (PLA glycerol)

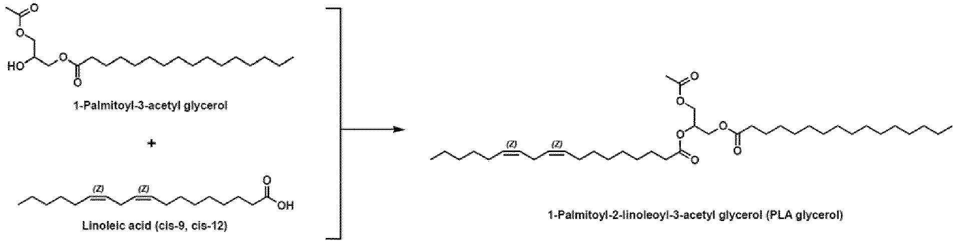

PLA glycerol was synthesized in the same manner as in Example 1, except that linoleic acid (cis-9,cis-12) was used instead of conjugated linoleic acid (cis-9,trans-11/trans-10,cis-12).

14.0g of linoleic acid (cis-9, cis-12) and 10.8g of triethylamine were added to 180ml of n-hexane, cooled to 0°C, and 7.0g of pivaloyl chloride was slowly added dropwise. After completion of the dropwise addition, the mixture was stirred at the same temperature for 1 hour, and then 18.0 g of 1-palmitoyl-3-acetyl glycerol and 1.0 g of 4-dimethylaminopyridine were added, and then stirred at room temperature for 10 hours. 180 ml of purified water was added, the layers were separated and the organic layer was dehydrated with MgSO₄, concentrated in vacuo, and purified by silica gel column (eluent: n-Hx:EA=20:1, v/v) to give 24.9 g (yield: 81%) of the target compound.

The anti-inflammatory activity experiments of the compounds synthesized in Examples 1 and 2 and Comparative Example were evaluated in the following animal experiments.

### A. Experimental animals

As experimental animals, 8-week-old C57BL/6 mice were bred for 7 days under the environment of a temperature of 22±2°C, a relative humidity of 65±5% and a 12-hour cycle in light and dark, and then adapted to the laboratory environment, and then used for the experiment. Solid feed (Samyang feed) and water were sufficiently supplied.

### B. Isolation and culture of peritoneal macrophage

HBSS was intraperitoneally injected into a mouse to extract macrophages, which was centrifuged at 3,000 rpm for 5 minutes, and then 100 units/mL of penicillin/streptomycin was added to DMEM medium with 10% fetal bovine serum (FBS), and peritoneal macrophage was isolated. It was incubated in a 37 °C, 5% CO2 incubator for 24 hours, and then used in the experiment.

### Experimental Example 1: Evaluation of cytotoxicity using MTT assay

100 uL of the cultured peritoneal macrophages were dispensed into a 96-well plate at 3 × 10⁵ cells/well and cultured overnight. After removing the medium, the peritoneal macrophage was treated with the compounds of Example 1 (PCA glycerol) and Example 2 (CPA glycerol) at different concentrations (10 µg/ml, 100 µg/ml, and 200 µg/ml, respectively), and then incubated for 24 hours in a 37°C, 5%CO₂ incubator. After incubation, the medium was removed, 40uL of MTT (5mg/mL) reagent was dispensed and incubated for 4 hours in a CO2 incubator, the MTT reagent was removed, and 600uL of DMSO reagent was dispensed, and then left at room temperature for 30 minutes. Then, the absorbance (OD) was measured at 540 nm with a microplate reader.

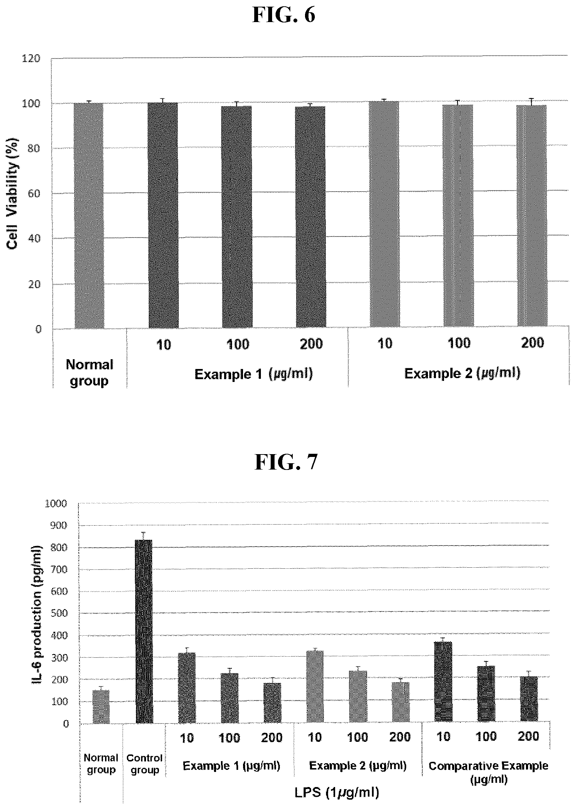

The cell viability was measured by the MTT assay after treatment with the compounds of Examples 1 and 2, and shown in Table 2 and the graphs of FIG. 6 below. As shown in Table 2 and FIG. 6, it can be seen that the cell viability does not significantly differ even when treated at a maximum of 200 µg/ml. Therefore, it was confirmed that the novel compound according to the present disclosure has no cytotoxicity.

**[Table 2]**

| | Normal group | Example 1 (µg/ml) | | | Example 2 (µg/ml) | | |
|---|---|---|---|---|---|---|---|
| | | 10 | 100 | 200 | 10 | 100 | 200 |
| Cell viability (%) | 100±1.2 | 100±2.1 | 98.3±1.9 | 98.1±1.2 | 100±0.9 | 98.3±1.7 | 98.0±2.8 |

### Experimental Example 2: Anti-inflammatory activity experiment (evaluation of inhibition of IL-6, IL-1β and TNF-α production)

The secretion amount of IL-6, IL-1β and TNF-α secreted from mouse peritoneal macrophages induced by lipopolysaccharide (LPS), which is an inflammatory response inducer, was measured by an ELISA assay (Millipore, USA) to thereby evaluate the anti-inflammatory activity.

In order to obtain a cell culture solution, mouse peritoneal macrophages were adjusted to 3 × 10⁵ cells/mL, inoculated into a 96-well plate, cultured for 24 hours, and then the compounds of Example 1 (PCA glycerol), Example 2 (CPA glycerol) and Comparative Example (PLA glycerol) were treated at different concentrations (10 µg/ml, 100 µg/ml, and 200 µg/ml, respectively), and LPS (1 µg/ml) was treated. The normal group was untreated, and the control group was treated with only LPS (1µg/ml) on peritoneal macrophages. After culturing for 12 hours, a supernatant was obtained by centrifugation. ELISA was run in microplates coated with anti-mouse IL-6, IL-1β and TNF-α as the capture antibody. Then, the cells were washed with phosphate buffered saline (PBST) containing 0.05% Tween 20, blocked with 10% FBS, and washed with PBST. The cell culture supernatant was dispensed into the wells, and reacted at room temperature for 2 hours. After the reaction, washing was performed with PBST and dilute biotinylated anti-mouse IL-6, IL-1β and TNF-α detection antibody and streptavidin-horseradish peroxydase conjugate were dispensed and reacted at room temperature for 1 hour. Then, washing was performed again with PBST, and an OPD solution was added, and then the mixture was subjected to dark reaction at room temperature for 30 minutes. After the reaction was terminated with 2N H₂SO₄, the absorbance was measured at 450 nm using a microplate reader.

The amount of IL-6 production of the compounds of Example 1 and Example 2 were measured and shown in Table 3 and the graph of FIG. 7 below. As shown in Table 3 and FIG. 7, it was confirmed that the compounds prepared in Examples 1 and 2 significantly reduced in the amount of IL-6 production as compared with the control group, and also exhibited significant IL-6 production inhibitory effects even in comparison with the Comparative Example.

**[Table 3]**

| | | LPS (1µg/ml) | | | | | | | | | |
|---|---|---|---|---|---|---|---|---|---|---|---|
| | Normal group | Control group | Example 1 (µg/ml) | | | Example 2 (µg/ml) | | | Comparative Example (µg/ml) | | |
| | | | 10 | 100 | 200 | 10 | 100 | 200 | 10 | 100 | 200 |
| IL-6 producti on amount (pg/ml) | 150±18 | 834±32 | 321±22 | 225±23 | 180±25 | 325±13 | 234±20 | 179±17 | 364±16 | 251±24 | 203±25 |

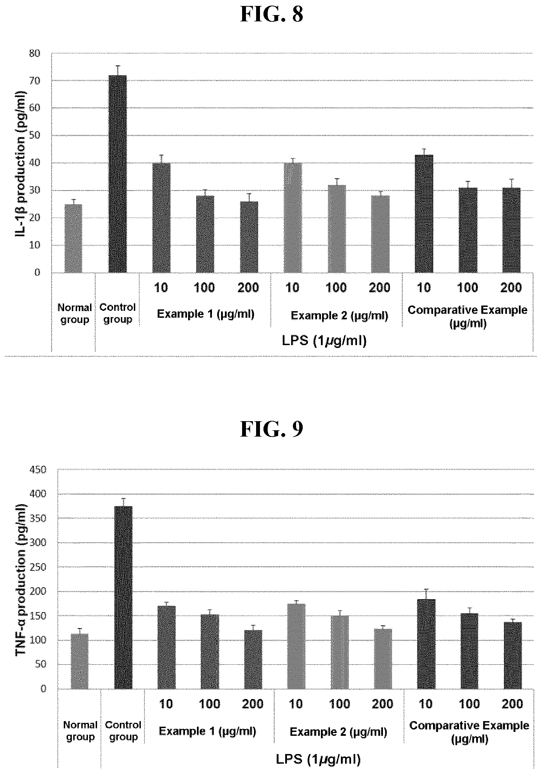

The amount of IL-1β production of the compounds of Examples 1 and 2 was measured, and shown in Table 4 and the graph of FIG. 8 below. As shown in Table 4 and FIG. 8, it was confirmed that the compounds prepared in Examples 1 and 2 significantly decreased in the amount of IL-1β production compared to the control group, and also exhibited significant inhibitory effects on IL-1β production even in comparison with the Comparative Example.

**[Table 4]**

| | | LPS (1µg/ml) | | | | | | | | | |
|---|---|---|---|---|---|---|---|---|---|---|---|
| | Normal group | Control group | Example 1 (µg/ml) | | | Example 2 (µg/ml) | | | Comparative Example (µg/ml) | | |
| | | | 10 | 100 | 200 | 10 | 100 | 200 | 10 | 100 | 200 |
| IL-1β Producti on amount (pg/ml) | 25±1.8 | 72±3.5 | 40±2.9 | 28±2.3 | 26±2.8 | 40±1.7 | 32±2.4 | 28±1.7 | 43±2.2 | 31±2.4 | 31±3.1 |

The amount of TNF-α production of the compounds of Examples 1 and 2 was measured, and shown in Table 5 and the graph of FIG. 9 below. As shown in Table 5 and FIG. 9, it was confirmed that the compounds prepared in Examples 1 and 2 significantly reduced in the amount of TNF-α production compared to the control group, and also exhibited significant inhibitory effects on TNF-α production even in comparison with the Comparative Example.

**[Table 5]**

| | | LPS(1µg/ml) | | | | | | | | | |
|---|---|---|---|---|---|---|---|---|---|---|---|
| | Normal group | Control group | Example 1 (µg/ml) | | | Example 2 (µg/ml) | | | Comparative Example (µg/ml) | | |
| | | | 10 | 100 | 200 | 10 | 100 | 200 | 10 | 100 | 200 |
| TNF-α producti on amount | 113±11 | 375±16 | 170±8 | 153±11 | 121±10 | 175±7 | 151±10 | 123±7 | 184±21 | 155±12 | 137±7 |
| (pg/ml) | | | | | | | | | | | |

### Experimental Example 3: Anti-inflammatory activity experiment (evaluation of inhibition of NO production)

The cultured peritoneal macrophages were suspended in DMEM containing 10% FBS, dispensed into a 96-well plate at 5 × 10⁵ cells/well and incubated for 24 hours in a 37°C, 5% CO₂ incubator, and replaced with new DMEM medium. Then, the peritoneal macrophages were treated with each of the compounds of Examples 1 and 2 and Comparative Example at different concentrations (10 µg/ml, 100 µg/ml, and 200 µg/ml, respectively), and LPS (1 µg/ml) was treated. Then, the cells were incubated for 24 hours. After incubation, the supernatant was separated, centrifuged at 3000 rpm for 5 minutes, and the separated supernatant was dispensed onto a new microplate. The normal group was untreated, and the control group was treated with only LPS (1µg/ml) on the peritoneal macrophages.

The same amount of Griess reagent (1% sulfanilamide, 0.1% naphthyl-ethylenediamine dihydrochloride, 2% phosphoric acid) was treated, and reacted at room temperature for 10 minutes. After the culture solution was reacted with the Griess solution for 5 minutes, the absorbance (OD) was measured at 540 nm.

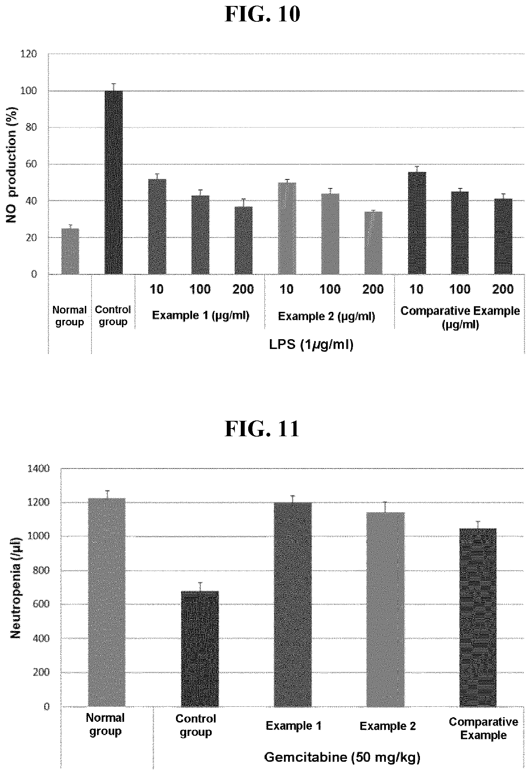

FIG. 10 is a graph showing the NO production inhibitory effect of the novel compounds of Examples 1 and 2 of the present disclosure.

The nitrogen monoxide (NO) production rates of the compounds of Examples 1 and 2 were measured, and shown in Table 6 and the graph of FIG. 10 below. As shown in Table 6 and FIG. 10, it was confirmed that the compounds prepared in Examples 1 and 2 significantly reduced in the NO production rate compared to the control group, and also exhibited significant inhibitory effects on the NO production even in comparison with the Comparative Example.

**[Table 6]**

| | | LPS (µg/ml) | | | | | | | | | |
|---|---|---|---|---|---|---|---|---|---|---|---|
| | Control group | Control group | Example 1 (µg/ml) | | | Example 2 (µg/ml) | | | Comparative Example (µg/ml) | | |
| | | | 10 | 100 | 200 | 10 | 100 | 200 | 10 | 100 | 200 |
| NO producti on rate (%) | 25±2 | 100±4 | 52±3 | 43±3 | 37±4 | 50±2 | 44±3 | 34±1 | 56±3 | 45±2 | 41±3 |

The compounds according to the present disclosure can be used for the prevention or treatment of inflammatory disease caused by inflammatory cytokines, for example, inflammatory diseases selected from the group consisting of atopic dermatitis, edema, dermatitis, allergy, asthma, conjunctivitis, periodontitis, rhinitis, otitis media, sore throat, tonsillitis, pneumonia, gastritis, colitis, gout, hepatic spondylitis, fibromyalgia, psoriatic arthritis, osteoarthritis, rheumatoid arthritis, shoulder periarthritis, myositis, hepatitis, cystitis, and nephritis.

PLA glycerol in Comparative Example is EC-18, a candidate substance for antineoplastic chemotherapy-induced neutropenia (CIN), and is known to have recently completed Phase II clinical trials of the FDA. It has been reported to inhibit neutropenia by a mechanism of action different from that of the conventional therapeutic agent G-CSF (filgrastim) or its derivatives (pegfilgrastim).

In the following, the inhibitory effect of the compounds of Examples 1 and 2 of the present invention and the compounds of Comparative Examples on neutropenia induced by anticancer chemotherapy agents is verified by a mouse experiment.

### Experimental Example 4: Mouse experiment of antineoplastic chemotherapy-induced neutropenia

50 mg/kg of gemcitabine, used as an anticancer agent, was injected intraperitoneally into C57BL/6 mice every day for 3 weeks and used as a control group. 50 mg/kg of the compounds of Example 1, Example 2, and Comparative Example 1 were orally administered for 3 weeks every day, and after 3 weeks, the number of neutrophils in 1 µl of blood was measured using an automatic blood sample analyzer (Auto Hematology Analyzer BC-5900, Mindray), and the results are shown in Table 7 and FIG. 11 below.

**[Table 7]**

| | - | Gemcitabine (50mg/kg) | | | |
|---|---|---|---|---|---|
| | Normal group | Control group | Example 1 | Example 2 | Comparative Example |
| Number of neutrophils (/µℓ) | 1224±45 | 678±54 | 1198±43 | 1142±62 | 1047±42 |

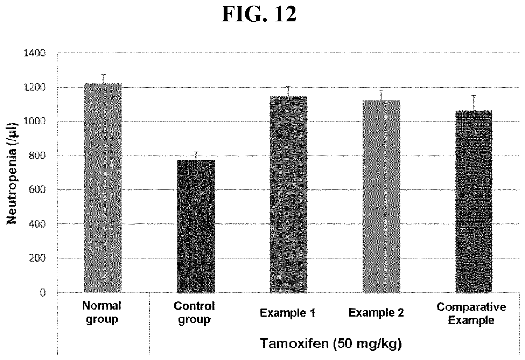

As shown in Tables 7 and FIG. 11, the mice treated with gemcitabine showed an about 45% reduction in the number of neutrophils, and the compound of Comparative Example (PLA glycerol) showed a 14.5% reduction in the number of neutrophils, whereas the compound of Example 1 (PCA glycerol) showed an about 2% reduction in the number of neutrophils, and the compound of Example 2 (CPA glycerol) showed a 6.7% reduction in the number of neutrophils, confirming that there was a significant effect in comparison with the Comparative Example as well as the control group. 50 mg/kg of Tamoxifen, used as an anticancer agent, was intraperitoneally injected into C57BL/6 mice every day for 3 weeks and used as a control group, and 50 mg/kg of the compounds of Example 1, Example 2, and Comparative Example 1 were orally administered for 3 weeks every day, and after 3 weeks, the number of neutrophils in 1 µl of blood was measured using an automatic blood sample analyzer (Auto Hematology Analyzer BC-5900, Mindray), and the results are shown in Table 8 and FIG. 12 below.

**[Table 8]**

| | - | Tamoxifen (50mg/kg) | | | |
|---|---|---|---|---|---|
| | Normal group | Control group | Example 1 | Example 2 | Comparative Example |
| Number of neutrophils (/µℓ) | 1224±45 | 774±49 | 1146±63 | 1124±57 | 1066±88 |

As shown in Table 8 and FIG. 12, the mice treated with tamoxifen showed an about 37% decrease in number of neutrophils, and the compound of Comparative Example (PLA glycerol) showed a 12.9% decrease in the number of neutrophils, whereas the compound of Example 1 (PCA glycerol) showed about 6.3% decrease in the number of neutrophils, and the compound of Example 2 (CPA glycerol) showed about 8.2% decrease in the number of neutrophils, confirming that there was a significant effect in comparison with the Comparative Example as well as the control group.

As the compounds of the present disclosure, known anticancer chemotherapy drugs can be effectively used as an inhibitory drug for neutropenia caused by Cyclophosphamide, Imatinib, Lenalidomide, Bortezomib, Pemetrexed, Methotrexate, Paclitaxel, Etoposide, Topotecan, irinotecan, Mechlorethanime, Chlorambucil, Melphalan, Carmustine (BCNU), Lomustine (CCNU), Ifosfamide, Procarbazine, Dacarbazine (DTIC), Altretamine, Mesna, Cisplatin, Carboplatin, Actinomycin D, Doxorubicin, Daunorubicin, 6-Mercaptopurine, 6-Thioguanine, Idarubicin, Epirubicin, Mitoxantrone, Azathioprine, 2-Chlorodeoxyadenosine, Hydroxyurea, 5-Fluorouracil, Cytosine arabinoside, Azacytidine, Fludarabine phosphate, Vincristine, Vinblastine, Vinorelbine, Docetaxel, Nab-paclitaxel, Dasatinib, Sunitinib, and the like, in addition to the above-mentioned anticancer chemotherapeutic drugs.

### INDUSTRIAL APPLICABILITY

The present disclosure relates to a novel compound isolated from *Cervi Parvum Cornu* and pharmaceutical uses thereof, and further, a chemical synthesis method of the novel compound.

## Claims

1. A pharmaceutical composition for the prevention or treatment of inflammatory diseases, leukopenia or neutropenia, comprising as an active ingredient at least one selected from compounds represented by the following Formulas 1 to 4.

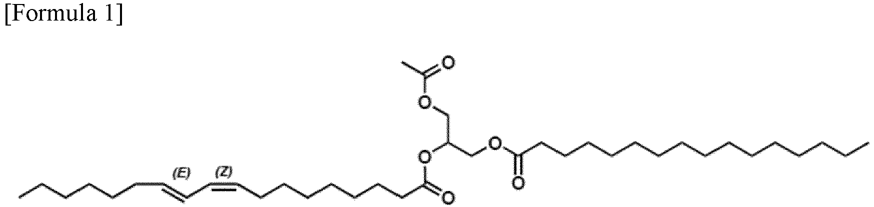

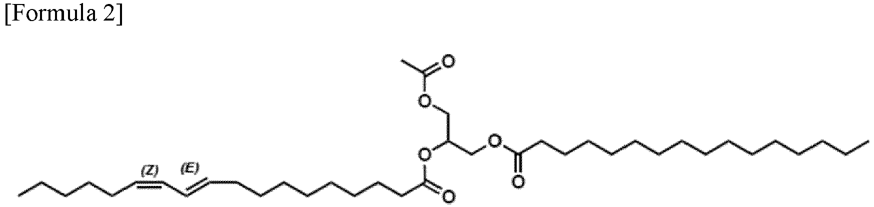

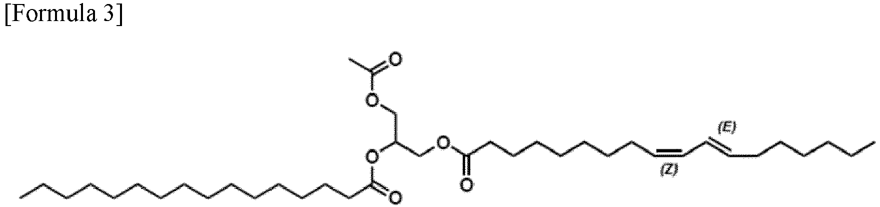

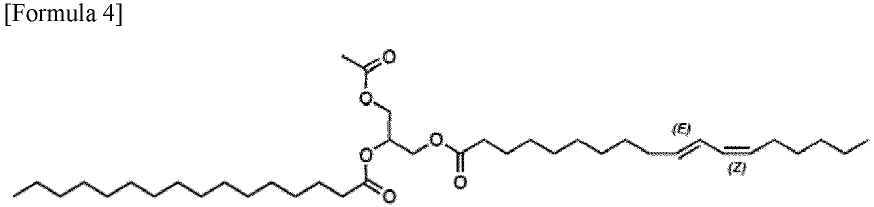

2. The pharmaceutical composition for the prevention or treatment of inflammatory diseases, leukopenia or neutropenia as set forth in claim 1,
wherein the active ingredient is a mixture of the compound represented by Formula 1 and the compound represented by Formula 2.

3. The pharmaceutical composition for the prevention or treatment of inflammatory diseases, leukopenia or neutropenia as set forth in claim 1,
wherein the active ingredient is a mixture of the compound represented by Formula 3 and the compound represented by Formula 4.

4. The pharmaceutical composition for the prevention or treatment of inflammatory diseases, leukopenia or neutropenia as set forth in claim 1,
wherein the active ingredient inhibits the production of inflammatory cytokines.

5. The pharmaceutical composition for the prevention or treatment of inflammatory diseases, leukopenia or neutropenia as set forth in claim 1,
wherein the inflammatory disease is selected from the group consisting of atopic dermatitis, edema, dermatitis, allergy, asthma, conjunctivitis, periodontitis, rhinitis, otitis media, sore throat, tonsillitis, pneumonia, gastritis, colitis, gout, hepatic spondylitis, fibromyalgia, psoriatic arthritis, osteoarthritis, rheumatoid arthritis, shoulder periarthritis, myositis, hepatitis, cystitis, and nephritis.

6. The pharmaceutical composition for the prevention or treatment of inflammatory diseases, leukopenia or neutropenia as set forth in claim 1,
wherein the neutropenia disease is neutropenia induced by anticancer chemotherapy drugs.

7. A health functional food for preventing or improving inflammatory diseases comprising compounds represented by the following Formulas 1 to 4 as an active ingredient

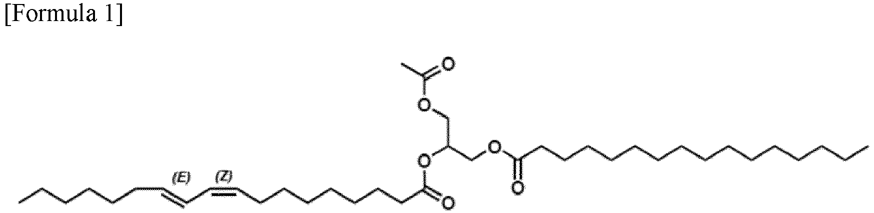

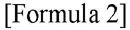

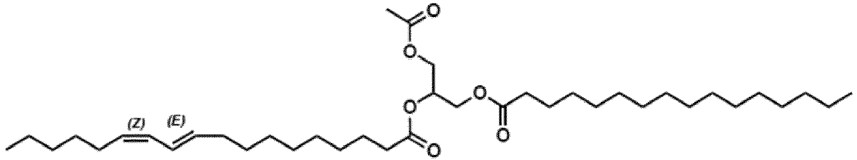

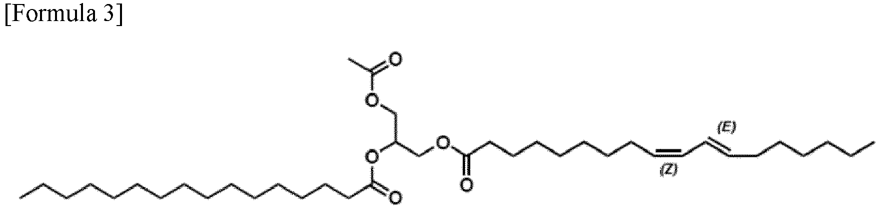

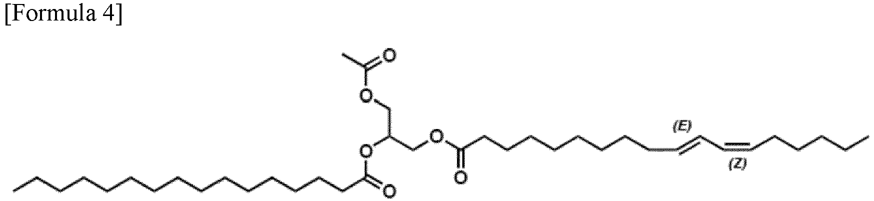

8. A method for synthesizing 1-palmitoyl-2-conjugated linoleoyl-3-acetyl glycerol (PCA glycerol) comprising the steps of:
(a) reacting palmitic acid with a compound represented by the following Formula 5 under basic conditions to synthesize 1-palmitoyl glycerol;
(b) reacting the 1-palmitoyl glycerol with acetyl halide under basic conditions to synthesize 1-palmitoyl-3-acetyl glycerol; and
(c) reacting the 1-palmitoyl-3-acetyl glycerol with conjugated linoleic acid under basic conditions. 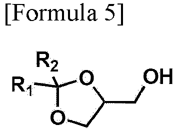 (wherein, R₁ and R₂ are each independently a C₁ ∼ C₃ alkyl group.)

9. The method for synthesizing 1-palmitoyl-2-conjugated linoleoyl-3-acetyl glycerol (PCA glycerol) as set forth in claim 8,
wherein in step 1, the palmitic acid is activated by adding pivaloyl halide, or
in step (c), the conjugated linoleic acid is activated by adding pivaloyl halide, and the halide is Cl, Br or I.

10. The method for synthesizing 1-palmitoyl-2-conjugated linoleoyl-3-acetyl glycerol (PCA glycerol) as set forth in claim 8,
wherein the conjugated linoleic acid is a mixture of conjugated linoleic acid (cis-9,trans-11) and conjugated linoleic acid (trans-10,cis-12).

11. A method for synthesizing 1-conjugated linoleoyl-2-palmitoyl-3-acetyl glycerol (CPA glycerol) comprising the steps of:
(a) reacting conjugated linoleic acid with a compound represented by the following Formula 5 under basic conditions to synthesize 1-conjugated linoleoyl-glycerol;
(b) reacting the 1-conjugated linoleoyl-glycerol with acetyl halide under basic conditions to synthesize 1-conjugated linoleoyl-3-acetyl glycerol; and
(c) reacting the 1-conjugated linoleoyl-3-acetyl glycerol with palmitic acid under basic conditions. 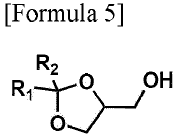 (wherein, R₁ and R₂ are each independently a C₁ ∼ C₃ alkyl group.)

12. The method for synthesizing 1-conjugated linoleoyl-2-palmitoyl-3-acetyl glycerol (CPA glycerol) as set forth in claim 11,
wherein in step (a), the conjugated linoleic acid is activated by adding pivaloyl halide, or in step (c), the palmitic acid is activated by adding pivaloyl halide, and
the halide is Cl, Br or I.

13. The method for synthesizing 1-conjugated linoleoyl-2-palmitoyl-3-acetyl glycerol (CPA glycerol) as set forth in claim 11,
wherein the conjugated linoleic acid is a mixture of conjugated linoleic acid (cis-9,trans-11) and conjugated linoleic acid (trans-10,cis-12).
